# EUROPÄISCHE PATENTSCHRIFT

(11) **EP 1 221 437 B1**
(45) Veröffentlichungstag und Bekanntmachung des Hinweises auf die Patenterteilung: **06.07.2005**
(21) Anmeldenummer: 02000129.3
(22) Anmeldetag: 04.01.2002
(51) Int. Cl.: C07C 209/48, C07C 213/02

(54) **Verfahren zur Herstellung von primären und sekundären Aminen aus Nitrilen**
Process for the preparation of primary and secondary amines from nitriles
Procédé de préparation d'amines primaires et secondaires à partir de nitriles

(30) Priorität: 05.01.2001 DE 10100313
(43) Veröffentlichungstag der Anmeldung: 10.07.2002
(73) Patentinhaber: BASF AKTIENGESELLSCHAFT, 67056 Ludwigshafen (DE)
(72) Erfinder: Pfeffinger, Joachim, 67063 Ludwigshafen (DE); Hüllmann, Michael, Dr., 64625 Bensheim (DE); Höhn, Arthur, Dr., 67281 Kirchheim (DE); Funke, Frank, Dr., 68159 Mannheim (DE); Ohlbach, Frank, Dr., 40219 Düsseldorf (DE); Gerlach, Till, Dr., 67071 Ludwigshafen (DE)
(74) Vertreter: Isenbruck, Günter, Dr.

(56) Entgegenhaltungen:
- EP-A- 0 316 761
- EP-A- 0 913 388
- DE-A- 3 935 641
- JIRI KRUPKA ET AL.: "Hydrogenation of 3-(Dimethylamino)propionitrile over Palladium Catalysts" COLLECTION OF CZECHOSLOVAK CHEMICAL COMMUNICATIONS., Bd. 65, Nr. 11, November 2000 (2000-11), Seiten 1805-1819, XP002195208 PRAGUE CS

## Beschreibung

Die Erfindung betrifft ein Verfahren zur Herstellung von Gemischen aus primären und sekundären Aminen aus Nitrilen an einem Palladium-Katalysator.

Verfahren zur Herstellung von Aminen aus Nitrilen an bestimmten PalladiumKatalysatoren sind bekannt.

In der DE-A-39 35 641 ist die Herstellung von Aminen aus 3-(Dimethylamino)propionitril (DMAPN) beschrieben. Als Produkte werden Bis-(3-(dimethylamino)propyl)amin (Bis-DMAPA) und Tris-(3-(dimethylamino)propyl)-amin (Tris-DMAPA) gebildet. Die Umsetzung wird dabei an Palladium auf Aluminiumoxid als Katalysator durchgeführt. Der Anteil an Bis-DMAPA beträgt maximal 32%.

In Coll. Czech. Chem. Comm. 65, 1805 -1819 (2000) wird die Hydrierung von 3-(Dimethylamino)propionitril über Palladiumkatalysatoren beschrieben. Die Umsetzung liefert vorzugsweise Gemische aus sekundären und tertiären Aminen.

EP-A- 0 869 113 betrifft ein Verfahren zur Herstellung tertiärer Amine aus Nitrilen und sekundären Aminen. Dabei werden sekundäre Amine in Gegenwart von Wasserstoff an einem Katalysator umgesetzt, der 0,9% Pd und 0,1% Pt auf Zirkondioxid als Träger enthält.

WO 99/32429 betrifft ein Verfahren zur Herstellung sekundärer Amine aus Nitrilen und primären Aminen. Die Umsetzung erfolgt dabei in Gegenwart von Wasserstoff ebenfalls am vorgenannten Katalysator aus 0,9% Pd und 0,1% Pt auf Zirkonoxid.

Aufgabe der vorliegenden Erfindung ist die Bereitstellung eines Verfahrens zur Herstellung von Gemischen aus primären Aminen und sekundären Aminen durch Umsetzung von Nitrilen mit Wasserstoff in Gegenwart von Katalysatoren.

Insbesondere sollen Gemische aus primären und symmetrisch aufgebauten sekundären Aminen hergestellt werden.

Die Aufgabe wird erfindungsgemäß gelöst durch ein Verfahren zur Herstellung von Gemischen aus primären Aminen der allgemeinen Formel (I)

X-CH₂-NH₂ (I)

und sekundären Aminen der allgemeinen Formel (II)

(X-CH₂-)₂ NH (II)

mit der Bedeutung
- X: ein gegebenenfalls durch C₁₋₂₀-Alkyl, C₃₋₈-Cycloalkyl,C₄₋₂₀-Alkylcycloalkyl, C₄₋₂₀-Cycloalkylalkyl, C₂₋₂₀-Alkoxyalkyl, C₆₋₁₄-Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy und/oder C₂₋₈-Alkoxycarbonyl substituiertes C₁₋₂₀-Alkyl, C₂₋₂₀-Alkenyl oder C₃₋₈-Cycloalkyl
durch Umsetzung von Nitrilen der allgemeinen Formel (III)

X-CN (III)

mit Wasserstoff bei Temperaturen von 50 bis 250°C und Drücken von 5 bis 350 bar in Gegenwart eines Pd enthaltenden Katalysators, wobei der Katalysator, bezogen auf das Gesamtgewicht des Katalysators, 0,1 bis 10 Gew.% Pd und zusätzlich 0,1 bis 10 Gew.-% mindestens eines weiteren Metalls, ausgewählt aus den Gruppen IB und VIII des Periodensystems, Cer und Lanthan auf einem Träger enthält.

Erfindungsgemäß wurde gefunden, daß die Verwendung eines wie vorstehend definierten Katalysators zur Umsetzung von Nitrilen mit Wasserstoff zu Gemischen aus primären und sekundären Aminen zu einer längeren Standzeit bzw. Langzeitstabilität des Katalysators führen kann.

Die erfindungsgemäß eingesetzten Katalysatoren enthalten, bezogen auf das Gesamtgewicht des Katalysators, 0,1 bis 10 Gew.-%, vorzugsweise 0,1 bis 5 Gew.-%, besonders bevorzugt 0,2 bis 1 Gew.-% Palladium und zusätzlich bezogen auf das Gesamtgewicht des Katalysators, 0,1 bis 10 Gew.-%, vorzugsweise 0,1 bis 5 Gew.-%, besonders bevorzugt 0,2 bis 1 Gew.-% mindestens eines weiteren Metalls, ausgewählt aus den Gruppen IB und VIII des Periodensystems der Elemente, Cer und Lanthan. Es kann ein weiteres Metall oder ein Gemisch mehrerer weiterer Metalle eingesetzt werden. Bevorzugt werden Kupfer, Platin und Gemische daraus, besonders bevorzugt Platin verwendet. Besonders bevorzugt ist ein Katalysator, der 0,2 bis 1 Gew.-% Palladium und 0,2 bis 1 Gew.-% Platin, insbesondere 0,3 bis 0,5 Gew.-% Palladium und 0,3 bis 0,5 Gew.-% Platin, bezogen auf das Gesamtgewicht des Katalysators, enthält.

Besonders bevorzugt ist ein Katalysator, der etwa 0,4 Gew.-% Pd und etwa 0,4 Gew.-% Pt, bezogen auf das Gesamtgewicht des Katalysators, auf ZrO₂ als Träger enthält.

Vorzugsweise weist der Katalysator nur Pd und Pt als Aktivkomponenten auf. Besonders bevorzugt besteht der Katalysator aus Pd und Pt auf dem Träger. Dabei liegen vorzugsweise in etwa oder genau gleiche Mengen an Pd und Pt vor, auf das Gewicht bezogen.

Als Träger können alle bekannten geeigneten Träger verwendet werden. Beispielsweise ist der Träger ausgewählt aus Aktivkohle, Siliciumcarbid und Metalloxiden. Dabei werden als Metalloxide vorzugsweise Aluminiumoxid, Siliciumdioxid, Titandioxid, Zirkoniumdioxid, Zinkoxid, Magnesiumoxid oder deren Gemische verwendet, die gegebenenfalls mit Alkali- und/oder Erdalkalimetalloxiden dotiert sind. Besonders bevorzugt werden γ-Aluminiumoxid, Siliciumdioxid, Zirkoniumdioxid oder Titanoxid oder Gemische davon eingesetzt, insbesondere ZrO₂. Die Träger können in beliebiger Form eingesetzt werden, beispielsweise als Extrudate (in Form von Strängen), Pellets oder Tabletten. Die Katalysatoren können nach allgemein bekannten Verfahren hergestellt werden, beispielsweise durch Tränken des Trägers mit Lösungen von Verbindungen der eingesetzten Metalle. Palladium kann beispielsweise durch Tränken des Trägers mit Lösungen von PdCl₂ oder Pd(NO₃)₂ aufgetragen werden.

Die Träger können beispielsweise mit Metall-Vorläufern beschichtet werden. Als Metallvorläufer eignen sich Metallsalze, wie Nitrate, Nitrosylnitrate, Halogenide, Carbonate, Carboxylate, Acetylacetonate, Chlorokomplexe, Nitritokomplexe und Aminkomplexe. Bevorzugt sind Nitrate, Chloride, Chlorokomplexe und Aminkomplexe. Das Aufbringen erfolgt vorzugsweise durch gemeinsames Fällen oder Tränken. Die Metallvorläufer der Metalle können gleichzeitig oder nacheinander aufgetränkt werden. Die Reihenfolge, in der die Aktivkomponenten aufgetränkt werden, ist beliebig wählbar.

Weitere Verfahren zur Herstellung der erfindungsgemäß verwendeten Katalysatoren sind dem Fachmann bekannt und schließen das Bedampfen, Besputtern und gemeinsame Fällen ein.

Die Oberfläche, das Porenvolumen und die Porengrößenverteilung des Katalysators sind in weiten Bereichen unkritisch.

Das erfindungsgemäße Verfahren wird bei Temperaturen von 50 bis 200°C, vorzugsweise 90 bis 170°C, besonders bevorzugt 120 bis 160°C und Drücken von 5 bis 300 bar, vorzugsweise 50 bis 250 bar, besonders bevorzugt 70 bis 210 bar diskontinuierlich oder bevorzugt kontinuierlich in Druckapparaturen wie Autoklaven oder bevorzugt in einem Rohrreaktor durchgeführt. Der Druck ist dabei vorzugsweise der Wasserstoffdruck im Reaktor. Bei der Verwendung eines Rohrreaktors kann der verwendete Katalysator auch als Festbettkatalysator vorliegen.

Die Umsetzung wird vorzugsweise in der Flüssigphase in Sumpf- oder Rieselfahrweise, besonders bevorzugt in Sumpffahrweise durchgeführt. Dabei wird insbesondere bevorzugt ohne Verwendung von Ammoniak gearbeitet.

Die Katalysatorbelastung, bezogen auf das eingesetzte Nitril, beträgt vorzugsweise 0,1 bis 2 kg/(l h), insbesondere etwa 0,1 kg/(l h). Dabei kann ein Teil des flüssigen Reaktionsaustrages in die Umsetzung zurückgeführt werden. Vorzugsweise wird mit einer Rückführung von flüssigem Reaktionsaustrag in einer Menge von 1 bis 20 kg/(l_{Kat} h), insbesondere 5 bis 10 kg/(l_{Kat} h) gearbeitet.

Das erfindungsgemäße Verfahren läßt sich lösungsmittelfrei oder in Lösungsmitteln, wie Wasser, Methanol, Ethanol, Tetrahydrofuran, Methyl-tert.-butylether oder N-Methylpyrrolidon durchführen. Im Lösungsmittel kann dabei das Nitril der allgemeinen Formel (III) gelöst sein. Das Lösungsmittel kann auch getrennt dem Reaktor an beliebiger Stelle zugeführt werden. Vorzugsweise wird lösungsmittelfrei gearbeitet.

Die im erfindungsgemäßen Verfahren erhaltenen Amine der allgemeinen Formeln (I) und (II) lassen sich in an sich bekannter Weise, beispielsweise destillativ, vom Reaktionsgemisch abtrennen und reinigen.

Beispielsweise ist es möglich, durch Rektifikation einen Strom mit reinem sekundärem Amin und einen Strom mit primärem Amin zu erhalten, wobei der Strom mit dem primären Amin in die Synthese zurückgeführt werden kann.

Erfindungsgemäß werden vorzugsweise die primären Amine der allgemeinen Formel (I) und die sekundären Amine der allgemeinen Formel (II) in einem Gewichtsverhältnis von 10 : 1 bis 1 : 10, vorzugsweise 2 bis 4 : 5 gebildet. Dabei werden vorzugsweise möglichst geringe Mengen an tertiären Aminen, insbesondere keine tertiären Amine gebildet. Vorzugsweise liegt die Menge an gebildeten tertiären Aminen unter 15 Gew.-%, besonders bevorzugt unter 10 Gew.-%, bezogen auf den Reaktionsaustrag (ohne Lösungsmittel).

Im erfindungsgemäßen Verfahren werden Nitrile der allgemeinen Formel (III) umgesetzt.

X-CN (III)

- X: ist dabei ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, C₆₋₁₄-Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy und/oder C₂₋₈-Alkoxycarbonyl substituiertes C₁₋₂₀-Alkyl, C₂₋₂₀-Alkenyl oder C₃₋₈-Cycloalkyl.

X ist dabei vorzugsweise C₁₋₁₂-, besonders bevorzugt C₁₋₈-, insbesondere C₁₋₆-, speziell C₁₋₄-Alkyl, das verzweigt oder unverzweigt sein kann und vorzugsweise unverzweigt ist. Beispiele sind unverzweigte Reste aus 1, 2, 3, 4, 5, 6, 7, 8, 9, 10, 11, 12, 13, 14, 15, 16, 17, 18, 19, 20 Methyleneinheiten, C(C)-C-C, C-C(C)-C, C-C(C)₂-C als Struktureinheiten. Bevorzugt sind als Struktureinheiten C, C-C, C-C-C, C-C-C-C, C-C-C-C-C-C, C-C(C)-C-C, C-C(C)-C-C, C-C-C(CN)-C-C-C, besonders bevorzugt C, C-C, C-C-C, C-C-C-C.

X kann, wie vorstehend angegeben, substituiert sein. Dabei kann die Anzahl der Substituenten bis zur Anzahl der substituierbaren Wasserstoffatome in X betragen. Abhängig von der Art des Restes können 1 bis 5, vorzugsweise 1 bis 3, insbesondere 0, 1 oder 2 Substituenten vorliegen. Als Substituenten kommen in Betracht:
- C₁₋₂₀-Alkoxy, bevorzugt C₁₋₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy wie Methoxy, Ethoxy, n-Propoxy, iso-Propoxy, n-Butoxy, iso-Butoxy, sec.-Butoxy und tert.-Butoxy,
- Hydroxy,
- C₁₋₂₀-Hydroxyalkyl, bevorzugt C₁₋₈-Hydroxyalkyl, besonders bevorzugt C₁₋₄-Hydroxyalkyl wie Hydroxymethyl, 1-Hydroxyethyl, 2-Hydroxyethyl, 2-Hydroxy-2-propyl und 3-Hydroxy-n-propyl,
- Amino,
- C₁₋₂₀-Alkylamino, bevorzugt C₁₋₈-Alkylamino besonders bevorzugt C₁₋₄-Alkylamino wie Methylamino, oder entsprechende Aminoalkyl, 1-Aminoethyl, 2-Aminoethyl, 2-Amino-n-propyl und 3-Amino-n-propyl,
- C₂₋₂₀-Dialkylamino, bevorzugt C₂₋₁₂-Dialkylamino, besonders C₂₋₈-Dialkylamino wie N,N-Dimethylamino, N,N-Diethylamino, N,N-Dipropylamino, N,N-Di-(1-methylethyl)amino, N,N-Dibutylamino, N,N-Di-(1-methylpropyl)-amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)-amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)-amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, n-(1-Methylethyl)-N-(1-methylpropyl)-amino, N-(1Methylethyl)-N-(1-methpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, n-(1,1-Dimethylethyl)-N-(1-methylpropyl)amino und N-(1,1-Diemthylethyl)-N-(2-methylpropyl)amino,
- C₃₋₁₂-Azacycloalkyl, bevorzugt C₃₋₈-Azacycloalkylamino, besonders bevorzugt C₅₋₈-Azacycloalkyl wie Pyrrolidin, Piperidin, Azepan, Piperazin, N-Alkylpiperazin und Morpholin,
- C₃₋₈-Cycloalkylamino wie Cyclopropylamino, Cyclobutylamino, Cyclopentylamino, Cyclohexylamino, Cycloheptylamino und Cyclooctylamino, bevorzugt Cyclopentylamino, Cyclohexylamino und Cyclooctylamino, besonders bevorzugt Cyclopentylamino und Cyclohexylamino,
- C₃₋₈-Dicycloalkylamino,
- Arylamino wie Phenylamino, 1-Naphthylamino und 2-Naphthylamino, bevorzugt Phenylamino,
- Aryl-C₁₋₈-alkylamino, bevorzugt Phenyl-C₁₋₈-alkylamino, besonders bevorzugt Phenyl-C₁₋₄-alkylamino wie Phenylmethylamino und Phenylethylamino,
- Halogen, bevorzugt Fluor, Chlor und Brom, besonders bevorzugt Fluor und Chlor,
- Mercapto,
- C₂₋₂₀-Oxacycloalkyl, bevorzugt C₂₋₈-Oxacycloalkyl, besonders bevorzugt C₂₋₈-Oxacycloalkyl, wie 2-Tetrahydrofuranyl, 3-Tetrahydrofuranyl, 2-Furanyl und 3-Furanyl,
- C₃₋₈-Cycloalkoxy wie Cyclopropoxy, Cyclobutoxy, Cyclopentoxy, Cyclohexoxy, Cycloheptoxy und Cyclooctoxy, bevorzugt Cyclopentoxy, Cyclohexoxy, besonders bevorzugt Cyclopentoxy und Cyclohexoxy,
- Aryloxy wie Phenoxy, 1-Naphthoxy und 2-Naphthoxy, bevorzugt Phenoxy.

Vorzugsweise liegen 0, 1 oder 2 Substituenten vor, die OH oder C₂₋₁₂-, vorzugsweise C₂₋₆-, insbesondere C₂₋₄-Dialkylamino sind. Insbesondere sind die Substituenten Dimethylamino oder OH.

Bevorzugte Nitrile der allgemeinen Formel (III) sind Acetonitril, Propionitril, Isopropionitril, Butyronitril, Valeronitril, Pentensäurenitril, Retensäurenitril, 3-Hydroxypropionitril, 3-Methoxypropionitril, 3-Ethoxypropionitril, 3-Propoxypropionitril, 3-Isopropoxypropionitril, 3-Cyclohexoxypropionitril, 2-Methyl-3-hydroxypropionitril, 3-Methoxy-2-methylpropionitril, 3-Ethoxy-2-methylpropionitril, 2-Methyl-3-propoxypropionitril, 3-Isopropoxy-2-methylpropionitril, 3-Cyclohexoxy-2-methylpropionitril, 3-Methyl-3-Hydroxypropionitril, 3-Methoxy-3-methylpropionitril, 3-Ethoxy-3-methylpropionitril, 3-Methyl-3-propoxypropionitril, 3-Isopropoxy-3-methylpropionitril, 3-Cyclohexoxy-3-methylpropionitril, 3-Aminopropionitril, 3-Methylaminopropionitril, 3-Dimethylaminopropionitril, 3-Ethylaminopropionitril, 3-Diethylaminopropionitril, 3-Propylaminopropionitril, 3-Dipropylaminopropionitril, 3-Isopropylaminopropionitril, 3-Diisopropylaminopropionitril, 3-Cyclohexylaminopropionitril, 3-Dicyclohexylaminopropionitril, N-(Cyanoethyl)-N-methylanilin. Besonders bevorzugt sind 3-Hydroxypropionitril, 3-Methoxypropionitril, 3-Dimethylaminopropionitril, 3-Diethylaminopropionitril, 3-Cyclohexylaminopropionitril und 3-Methylaminopropionitril, insbesondere Biscyanethylether, Biscyanethylamin, N-Methyl-biscyanethylamin, N-Ethyl-biscyanethylamin, N-n-Propyl-biscyanethylamin, N-n-Propyl-biscyanethylamin, Polyisobutylennitril, N-Polyisobutylenaminopropionitril, Triscyanethylamin, 5-Aminovaleriansäurenitril, 5-Methylaminovaleriansäurenitril, 5-Dimethylaminovaleriansäurenitril, 6-Aminocapronsäurenitril, 6-Methylaminocapronsäurenitril, 6-Dimethylaminocapronsäurenitril, 5-Amino-4-methylvaleriansäurenitril, 5-Methyl-amino-4-methylvaleriansäurenitril, 5-Dimethylamino-4-methylvaleriansäurenitril, 5-Ethylamino-4-methylvaleriansäurenitril, 5-Diethylamino-4-methylvaleriansäurenitril, 5-Amino-2-methylvaleriansäurenitril, 5-Methylamino-2-methylvaleriansäurenitril, 5-Dimethylamino-2-valeriansäurenitril, 5-Ethylamino-2-methylvaleriansäurenitril, 5-Diethylamino-2-methylvaleriansäurenitril, 4-Cyanokorksäuredinitril, Acrylnitril.

Insbesondere bevorzugt sind Adipodinitril, 3-Dimethylaminopropionitril (DMAPN) und 3-Hydroxypropionitril, speziell DMAPN.

Die Amingemische, vorzugsweise DMAPA und Bis-DMAPA sind Härter für Epoxidharze, Katalysatoren für Polyurethane, Zwischenprodukte zur Herstellung quartärer Ammoniumverbindungen, Weichmacher, Korrosionsinhibitoren, Textilhilfsmittel, Farbstoffe und Emulgatoren. Mehrfach funktionalisierte tertiäre Amine dienen außerdem zur Herstellung von Kunstharzen, Ionenaustauschern, Pharmazeutika, Pflanzenschutz- und Schädlingsbekämpfungsmitteln.

Die Erfindung wird durch nachstehendes Beispiel näher erläutert.

### Beispiel

Ein elektrisch beheizter Rohrreaktor mit 30 mm Innendurchmesser, einem zentral angebrachten Thermoelement mit 12 mm Durchmesser und einer Gesamtlänge von 2,0 m wird mit einer Mischung aus 570 g (=500 ml) des Katalysators und 450 ml Edelstahlfüllkörpern gefüllt. Der Katalysator besteht aus 0,4 Gew.-% Pt und 0,4 Gew.-% Pd auf Zirkondioxid als Trägermaterial und hat die Form von Ringen mit 7 mm Durchmesser, 3 mm Dicke und einem Lochdurchmesser vom 3 mm.

Vor der Reaktion wird der Katalysator drucklos über einen Zeitraum von 12 Stunden mit reinem Wasserstoff bei 160°C reduziert.

Durch den Reaktor werden im Gleichstrom von unten nach oben 500 g/h an 3-(Dimethylamino)-propionitril (DMAPN), 4,0 kg/h an flüssigem Reaktionsaustrag und 0,38 Nm³/h Wasserstoff zugegeben. Die Edukte werden vor dem Reaktor auf eine Temperatur von 140°C erhitzt. Der Reaktor wird bei einer Temperatur von 140°C und einem Gesamtdruck von 80 bar gehalten.

Das aus dem Reaktor austretende Gemisch wird abgekühlt, ein Teil der Flüssigkeit wird an den Reaktoreingang zurückgeführt, und der übrige Teil wird auf Normaldruck entspannt. Mittels gaschromatographischer Analyse wurde im Reaktoraustrag ein Gehalt von 50 Gew.-% an Bis-(3-dimethylaminopropyl)-amin (Bis-DMAPA), 30 Gew.-% an 3-Dimethylaminopropylamin (DMAPA), 3,0 Gew.% an DMAPN sowie 17 Gew.% an verschiedenen Nebenprodukten gefunden. Der Umsatz an DMAPN lag damit bei 97%.

In einer Labordestillation mit Füllkörperschüttung (20 theoretischen Trennstufen) wurden anschließend 2300 g des flüssigen Reaktoraustrags bei einem Kopfdruck von 30 mbar (abs.) und einem Rücklaufverhältnis von 5 : 1 diskontinuierlich aufdestilliert. Es wurde eine Fraktion mit 730 g (Übergangstemperatur: 45°C) mit einem Gehalt von 90,0 Gew.-% an DMAPA, 5,5% an DMAPN und 4,5% an Nebenprodukten sowie eine zweite Fraktion mit 960 g (Übergangstemperatur: 125°C) mit einem Gehalt von 99,3 Gew.-% an Bis-DMAPA erhalten.

## Patentansprüche

1. Verfahren zur Herstellung von Gemischen aus primären Aminen der allgemeinen Formel (I)
X-CH₂-NH₂ (I)
und sekundären Aminen der allgemeinen Formel (II)
(X - CH₂-)₂ NH (II)
mit der Bedeutung
X ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, C₆₋₁₄-Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy und/oder C₂₋₈-Alkoxycarbonyl substituiertes C₁₋₂₀-Alkyl, C₂₋₂₀-Alkenyl oder C₃₋₈-Cycloalkyl
durch Umsetzung von Nitrilen der allgemeinen Formel (III)
X-CN (III)
mit Wasserstoff bei Temperaturen von 50 bis 250°C und Drücken von 5 bis 350 bar in Gegenwart eines Pd enthaltenden Katalysators, wobei der Katalysator, bezogen auf das Gesamtgewicht des Katalysators, 0,1 bis 10 Gew.% Pd und zusätzlich 0,1 bis 10 Gew.-% mindestens eines weiteren Metalls, ausgewahlt aus den Gruppen IB und VIII des Penodensystems, Cer und Lanthan auf einem Träger enthält.

2. Verfahren nach Anspruch 1, **dadurch gekennzeichnet, daß** der Katalysator 0,2 bis 1 Gew.% Pd und 0,2 bis 1 Gew.-% Pt enthält.

3. Verfahren nach Anspruch 1 oder 2, **dadurch gekennzeichnet, daß** der Träger ausgewählt ist aus Aktivkohle, Siliciumcarbid und Metalloxiden.

4. Verfahren nach Anspruch 3, **dadurch gekennzeichnet, daß** der Träger ZrO₂ ist.

5. Verfahren nach einem der Ansprüche 1 bis 4, **dadurch gekennzeichnet, daß** X hneares C₁₋₆-Alkyl mit bis zu 2 Substituenten ist.

6. Verfahren nach Anspruch 5, **dadurch gekennzeichnet, daß** die Substituenten C₂₋₁₂-Dialkylamino oder OH sind.

7. Verfahren nach Anspruch 6, **dadurch gekennzeichnet, daß** das Nitril der allgemeinen Formel (III) 3-(Dimethylammo)propionitril (DMAPN) ist, das zu einem Gemisch aus Bis-(3-(dimethylamino)propyl)amin (Bis-DMAPA) und 3-(Dimethylamino)propylamin (DMAPA) umgesetzt wird.

8. Verfahren nach einem der Ansprüche 1 bis 7, **dadurch gekennzeichnet, daß** die primären Amine der allgemeinen Formel (I) und die sekundären Amine der allgemeinen Formel (II) in einem Gewichtsverhältnis von 10 : 1 bis 1 : 10 gebildet werden.

9. Verwendung eines Katalysators, wie er in einem der Ansprüche 1 bis 4 definiert ist, zur Umsetzung von Nitrilen mit Wasserstoff zu primären und sekundären Aminen.

## Claims

1. A process for preparing mixtures of primary amines of the formula (I)
X-CH₂-NH₂ (I)
and secondary amines of the formula (II)
(X-CH₂-)₂NH (II)
where
X is C₁₋₂₀-alkyl, C₂₋₂₀-alkenyl or C₃₋₈-cycloalkyl which may be unsubstituted or substituted by C₁₋₂₀-alkyl, C₃₋₈-cycloalkyl, C₄₋₂₀-alkylcycloalkyl, C₄₋₂₀-cycloalkylalkyl, C₂₋₂₀-alkoxyalkyl, C₆₋₁₄-aryl, C₇₋₂₀-alkylaryl, C₇₋₂₀-aralkyl, C₁₋₂₀-alkoxy, hydroxy, C₁₋₂₀-hydroxyalkyl, amino, C₁₋₂₀-alkylamino, C₂₋₂₀-dialkylamino, C₂₋₁₂-alkenylamino, C₃₋₈-cycloalkylamino, arylamino, diarylamino, aryl-C₁₋₈-alkylamino, halogen, mercapto, C₂₋₂₀-alkenyloxy, C₃₋₈-cycloalkoxy, aryloxy and/or C₂₋₈-alkoxycarbonyl
by reacting nitriles of the formula (III)
X-CN (III)
with hydrogen at from 50 to 250°C and pressures of from 5 to 350 bar in the presence of a Pd-containing catalyst comprising, based on the total weight of the catalyst, from 0.1 to 10% by weight of Pd and additionally from 0.1 to 10% by weight of at least one additional metal selected from among groups IB and VIII of the Periodic Table, cerium and lanthanum on a support.

2. A process as claimed in claim 1, wherein the catalyst contains from 0.2 to 1% by weight of Pd and from 0.2 to 1% by weight of Pt.

3. A process as claimed in claim 1 or 2, wherein the support is selected from among activated carbon, silicon carbide and metal oxides.

4. A process as claimed in claim 3, wherein the support is ZrO₂.

5. A process as claimed in any of claims 1 to 4, wherein X is linear C₁₋₆-alkyl having up to 2 substituents.

6. A process as claimed in claim 5, wherein the substituents are C₂₋₁₂-dialkylamino or OH.

7. A process as claimed in claim 6, wherein the nitrile of the formula (III) is 3-(dimethylamino)propionitrile (DMAPN) which is converted into a mixture of bis(3-(dimethylamino)propyl)-amine (bis-DMAPA)and 3-(dimethylamino)propylamine (DMAPA).

8. A process as claimed in any of claims 1 to 7, wherein the primary amines of the formula (I) and the secondary amines of the formula (II) are formed in a weight ratio of from 10:1 to 1:10.

9. The use of a catalyst as defined in any of claims 1 to 4 for reacting nitriles with hydrogen to form primary and secondary amines.

## Revendications

1. Procédé pour la préparation de mélanges d'amines primaires de formule générale (I)
X - CH₂ - NH₂ (I)
et d'amines secondaires de formule générale (II)
(X - CH₂ -)₂ NH (II)
dans lesquelles
X signifie un groupe alkyle en C₁ à C₂₀, alcényle en C₂ à C₂₀ ou cycloalkyle en C₃ à C₈ le cas échéant substitué par un groupe alkyle en C₁ à C₂₀, cycloalkyle en C₃ à C₈, alkylcycloalkyle en C₄ à C₂₀, cycloalkylalkyle en C₄ à C₂₀, alcoxyalkyle en C₂ à C₂₀, aryle en C₆ à C₁₄, alkylaryle en C₇ à C₂₀, aralkyle en C₇ à C₂₀, alcoxy en C₁ à C₂₀, hydroxy, hydroxyalkyle en C₁ à C₂₀, amino, alkylamino en C₁ à C₂₀, dialkylamino en C₂ à C₂₀, alcénylamino en C₂ à C₁₂, cycloalkylamino en C₃ à C₈, arylamino, diarylamino, aryl(alkyle en C₁ à C₈)amino, halogène, mercapto, alcényloxy en C₂ à C₂₀, cycloalcoxy en C₃ à C₈, aryloxy et/ou alcoxycarbonyle en C₂ à C₈
par transformation de nitriles de formule générale (III)
X-CN (III)
avec de l'hydrogène à des températures de 50 à 250°C et des pressions de 5 à 350 bars en présence d'un catalyseur contenant Pd, le catalyseur contenant, par rapport au poids total du catalyseur, 0,1 à 10% en poids de Pd et en outre 0,1 à 10% en poids d'au moins un autre métal, choisi dans les groupes IB et VIII du système périodique, du cérium et du lanthane sur un support.

2. Procédé selon la revendication 1, **caractérisé en ce que** le catalyseur contient 0,2 à 1% en poids de Pd et 0,2 à 1% en poids de Pt.

3. Procédé selon la revendication 1 ou 2, **caractérisé en ce que** le support est choisi parmi le charbon actif, le carbure de silicium et les oxydes de métal.

4. Procédé selon la revendication 3, **caractérisé en ce que** le support est du ZrO₂.

5. Procédé selon l'une quelconque des revendications 1 à 4, **caractérisé en ce que** X est un groupe alkyle en C₁ à C₆ linéaire comprenant jusqu'à 2 substituants.

6. Procédé selon la revendication 5, **caractérisé en ce que** les substituants sont un groupe dialkylamino en C₂ à C₁₂ ou OH.

7. Procédé selon la revendication 6, **caractérisé en ce que** le nitrile de formule générale III est le 3-(diméthylamino)propionitrile (DMAPN), qui est transformé en un mélange de bis-(3-(diméthylamino)propyl)amine (Bis-DMAPA) et de 3-(diméthylamino)propylamine (DMAPA).

8. Procédé selon l'une quelconque des revendications 1 à 7, **caractérisé en ce que** les amines primaires de formule générale (I) et les amines secondaires de formule générale (II) sont formées dans un rapport pondéral de 10 : 1 à 1 : 10.

9. Utilisation d'un catalyseur tel qu'il est défini dans l'une quelconque des revendications 1 à 4 pour la transformation avec de l'hydrogène de nitriles en amines primaires et secondaires.
